(19) Europäisches Patentamt
European Patent Office
Office européen des brevets

(11) **EP 3 453 753 B1**

(12) **EUROPEAN PATENT SPECIFICATION**

(45) Date of publication and mention
of the grant of the patent:
**14.10.2020 Bulletin 2020/42**

(21) Application number: **17792708.4**

(22) Date of filing: **24.04.2017**

(51) Int Cl.:
*C12N 5/0735* (2010.01)   *C12N 5/071* (2010.01)
*C12N 5/074* (2010.01)   *C12M 3/00* (2006.01)
*C12N 5/00* (2006.01)

(86) International application number:
**PCT/JP2017/016249**

(87) International publication number:
**WO 2017/191775 (09.11.2017 Gazette 2017/45)**

(54) **METHOD FOR SUBCULTURING PLURIPOTENT STEM CELLS**

VERFAHREN ZUR SUBKULTIVIERUNG PLURIPOTENTER STAMMZELLEN

PROCÉDÉ DE SOUS-CULTURE DE CELLULES SOUCHES PLURIPOTENTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.05.2016 JP 2016093300**

(43) Date of publication of application:
**13.03.2019 Bulletin 2019/11**

(73) Proprietors:
• **FUJIFILM Corporation
Tokyo 106-8620 (JP)**
• **Kyoto University
Kyoto-shi, Kyoto 606-8501 (JP)**

(72) Inventors:
• **KAGAWA, Hideaki
Ashigarakami-gun
Kanagawa 258-8577 (JP)**
• **GOTO, Shun
Ashigarakami-gun
Kanagawa 258-8577 (JP)**
• **KOHASHI, Souichi
Ashigarakami-gun
Kanagawa 258-8577 (JP)**
• **NAKATSUJI, Norio
Kyoto-shi
Kyoto 606-8501 (JP)**
• **AIBA, Kazuhiro
Kyoto-shi
Kyoto 606-8501 (JP)**

(74) Representative: **Klunker IP
Patentanwälte PartG mbB
Destouchesstraße 68
80796 München (DE)**

(56) References cited:
WO-A1-2013/077423   WO-A1-2014/136581
JP-A- 2004 129 550   JP-A- 2010 526 530

• **TOMOMI?G. OTSUJI ET AL: "A 3D Sphere Culture
System Containing Functional Polymers for
Large-Scale Human Pluripotent Stem Cell
Production", STEM CELL REPORTS, vol. 2, no. 5,
1 May 2014 (2014-05-01), pages 734-745,
XP055171477, ISSN: 2213-6711, DOI:
10.1016/j.stemcr.2014.03.012**
• **YING NIE ET AL: "Scalable Passaging of
Adherent Human Pluripotent Stem Cells", PLOS
ONE, vol. 9, no. 1, 30 January 2014 (2014-01-30),
page e88012, XP055218521, DOI:
10.1371/journal.pone.0088012**
• **Ishita Chatterjee ET AL: "Induced Pluripotent
Stem (iPS) Cell Culture Methods and Induction of
Differentiation into Endothelial Cells" In:
"Methods in Molecular Biology", 17 February
2015 (2015-02-17), Humana Press, New York, NY,
US, XP055491923, ISSN: 1064-3745 vol. 1357,
pages 311-327, DOI: 10.1007/7651_2015_203,
*Whole document***

**Description**

Technical Field

The present disclosure relates to a method for subculturing pluripotent stem cells.

Background Art

For example, the following techniques are known as a technique relating to a method for subculturing pluripotent stem cells. For example, WO2013/077423A discloses a subculture method in which cell aggregations of uniform sized pluripotent stem cells which have an average diameter of about 200 μm to about 300 μm are divided through a mesh. In addition, WO2013/077423A discloses that the hole diameter of the mesh is about 50 μm and the divided cell aggregations have a diameter of about 80 μm to about 120 μm.

In addition, WO2014/136581A discloses that the flow rate of a liquid containing pluripotent stem cells passing through a mesh of a filter portion for subculture is set to 90 mL to 300 mL per minute.

TOMOMI G. OTSUJI et al.: "A 3D sphere culture system containing functional polymers for large-scale human pluripotent stem cell production", stem cell reports, vol.2 no. 5, 1 May 2014, pages 734 to 745 discloses a method for subculturing pluripotent stem cells, which comprises a culture step of culturing pluripotent stem cells to obtain a cell aggregation, and a dividing step of dividing the cell aggregation by passing the cell aggregation through a mesh-like film which has a plurality of holes. A mesh size of 50 μm was found optimal for subculture.

SUMMARY OF THE INVENTION

Technical Problem

For example, in order to treat liver diseases, heart diseases, or the like through cell transplantation, it is considered that a single patient requires transplantation of $1 \times 10^9$ or more differentiated cells. In order to realize this, it is indispensable to develop mass culture techniques for pluripotent stem cells.

For example, in culturing of pluripotent stem cells, in a case where sizes of cell aggregations (spheres) generated by culturing cells become too large, problems can occur; for example, the cell aggregations adhere to and are fused with each other, cells start to differentiate, or cells in central portions of the cell aggregations are necrotized. Accordingly, in order to prevent the sizes of cell aggregations from becoming too large, dividing processing for dividing the cell aggregations is necessary at an appropriate time during the cell culture period.

As disclosed in the above-described WO2013/077423A and WO2014/136581A, according to the subculture method in which a cell aggregation grown to a predetermined size is divided through a mesh, it is possible to improve the viability of subcultured cells compared to a subculture method through enzymatic treatment in the related art. However, in a case where it is attempted to realize mass culture on a scale of, for example, 100 liters, and in a case where the flow rate of a liquid containing pluripotent stem cells passing through the mesh is set to 90 mL to 300 mL per minute as disclosed in WO2014/136581A, it takes an enormous amount of time for treatment, and it is difficult to maintain homogeneity of cells. On the other hand, it is necessary to consider damage to the pluripotent stem cells for both a case where the flow rate of a liquid containing pluripotent stem cells passing through a mesh is increased and a case where the flow rate thereof is decreased.

The present disclosure provides a method for subculturing pluripotent stem cells suitable for mass culture.

Solution to Problem

A first aspect of the present disclosure is a method for subculturing pluripotent stem cells comprising: a culture step of culturing pluripotent stem cells to obtain a cell aggregation; and a dividing step of dividing the cell aggregation by passing the cell aggregation through a mesh-like film, which has a plurality of through-holes each having an opening dimension of 30 μm to 80 μm, at a speed of 15 cm/sec to 150 cm/sec.

In the culture step, the pluripotent stem cells may be cultured in a culture liquid containing a polymer compound. In the dividing step, it is preferable that an average value of circle-equivalent diameters of divided cell aggregations becomes 30 μm to 75 μm. The pluripotent stem cell may be an ES cell or an iPS cell.

Advantageous Effects of Invention

According to the above-described aspect of the present disclosure, it is possible to provide a method for subculturing pluripotent stem cells suitable for mass culture.

BRIEF DESCRIPTION OF THE DRAWINGS

Fig. 1A is a cross-sectional view showing a configuration of a dividing device according to an exemplary embodiment of the present disclosure.

Fig. 1B is a plan view showing a configuration of a mesh according to the exemplary embodiment of the present disclosure.

Fig. 2A is a micrograph showing states of cell aggregations immediately after division.

Fig. 2B is a micrograph showing states of cell aggregations after the lapse of 1 hour from the division.

Fig. 2C is a micrograph showing states of cell aggregations after the lapse of 2 hours from the division.

Fig. 2D is a micrograph showing states of cell aggregations after the lapse of 3 hours from the division.

Fig. 2E is a micrograph showing states of cell aggregations after the lapse of 4 hours from the division.

Fig. 2F is a micrograph showing states of cell aggregations after the lapse of 24 hours from the division.

Fig. 3 is a diagram showing a configuration of a cell culture device according to the exemplary embodiment of the present disclosure.

Fig. 4 is a diagram showing a flow of cells, a medium, and the like in a case where the cell culture device according to the exemplary embodiment of the present disclosure performs culture start processing.

Fig. 5 is a diagram showing a flow of cells, a medium, and the like in a case where the cell culture device according to the exemplary embodiment of the present disclosure performs medium replacement processing.

Fig. 6 is a diagram showing a flow of cells, a medium, and the like in a case where the cell culture device according to the exemplary embodiment of the present disclosure performs dividing processing.

Fig. 7 is a diagram showing a flow of cells, a medium, and the like in a case where the cell culture device according to the exemplary embodiment of the present disclosure performs freezing processing.

Fig. 8 is a flowchart showing a flow of processing in a cell culture program according to the exemplary embodiment of the present disclosure.

Fig. 9 is a sphere image after spheres are subcultured by being made to pass through a mesh (4) at a passage speed of 100 cm/s.

Fig. 10 is a sphere image after spheres are subcultured by being made to pass through a mesh (6) at a passage speed of 180 cm/s.

Fig. 11 is a sphere image after spheres are subcultured by being made to pass through a mesh (1) at a passage speed of 15 cm/s.

DESCRIPTION OF EMBODIMENTS

Hereinafter, an exemplary embodiment of the present disclosure will be described with reference to the drawings. In the drawings, the same or equivalent constituent elements and parts are denoted by the same reference numerals.

A method for subculturing pluripotent stem cells according to the exemplary embodiment of the present disclosure includes: a culture step of culturing pluripotent stem cells to obtain a cell aggregation; and a dividing step of dividing the cell aggregation by passing the cell aggregation through a mesh-like film, which has a plurality of through-holes each having an opening dimension of 30 $\mu$m to 80 $\mu$m, at a speed of 15 cm/sec to 150 cm/sec.

[Pluripotent Stem Cells]

Pluripotent stem cells applicable to the present exemplary embodiment are not particularly limited as long as these are undifferentiated cells which have a "self-replication ability" enabling cells to proliferate while maintaining an undifferentiated state and "pluripotency" enabling cells to be differentiated into all three germ layer lineages. Examples of pluripotent stem cells include embryonic stem cells (ES cells), induced pluripotent stem cells (iPS cells), embryonic germ cells (EG cells), embryonal carcinoma cells (EC cells), multipotent adult progenitor cells (MAP cells), adult pluripotent stem cells (APS cells), and multi-lineage differentiating stress enduring cells (Muse cells). ES cells and iPS cells are preferable as the pluripotent stem cells applicable to the present exemplary embodiment.

Animals from which pluripotent stem cells are derived are not particularly limited. Examples of mammals include humans, monkeys, mice, rats, dogs, cows, horses, and pigs. An animal (that is, a donor) from which pluripotent stem cells are derived is preferably an animal of the same species as an animal (that is, a recipient) into which the pluripotent stem cells or cells differentiated and induced from the pluripotent stem cells are to be transplanted. Establishment of pluripotent stem cells may be carried out through well-known method.

Whether pluripotent stem cells maintain an undifferentiated state can be confirmed through a well-known method. Examples thereof include a method of confirming expression of an undifferentiated marker through flow cytometry or immunostaining, and a method of confirming formation of a teratoma by subcutaneously injecting an immunodeficient mouse.

[Culture of Pluripotent Stem Cells]

Hereinafter, a culture step according to the exemplary embodiment of the present disclosure will be described. In the present exemplary embodiment, pluripotent stem cells preferably proliferate through suspension culture. Specifically, a culture container is filled with pluripotent stem cells and a medium, and the cells are subjected to suspension culture until an average value of circle-equivalent diameters of cell aggregations of the pluripotent stem cells becomes, for example, 200 μm to 300 μm. The circle-equivalent diameter refers to a diameter of a circle in a case where a region defined by each outline of extracted cell aggregations is regarded as a circle having the same area. By setting the average value of circle-equivalent diameters of the cell aggregations to be less than or equal to 300 μm, it is possible to suppress differentiation induction due to microenvironment formed by cytokines or the like secreted by cells. In addition, it is possible to suppress necrosis of cells occurring in a central portion of a cell aggregation and to suppress reduction in collection rate of living cells. On the other hand, by setting the average value of the circle-equivalent diameters of the cell aggregations to be greater than or equal to 200 μm, it is possible to make the collection rate of the cells be greater than or equal to a certain value. The sizes of the cell aggregations obtained in the culture step can be appropriately changed in consideration of suppression of differentiation induction, necrosis of cells, cell collection rate, and the like.

The medium applicable to the present exemplary embodiment is not particularly limited, and an example thereof includes any well-known medium for stem cell culture. Specific examples thereof include Dulbecco's Modified Eagle's Medium (DMEM), Dulbecco's Modified Eagle Medium: Nutrient Mixture F-12 (DMEM: F-12), Eagle's Minimal Essential Medium (EMEM), Basal Medium Eagle (BME), RPMI1640, MCDB104, MCDB153, 199, L15, mTeSR1, TeSR2, E8 (Nature Protocols 7: 2029-2040, 2012), and media obtained by adding cell proliferation factors to these media.

Various components usually added to a medium may be added to a medium, and examples thereof include: antibiotics such as penicillin and streptomycin; vitamins or vitamin derivatives such as ascorbic acid and retinoic acid; sugar sources such as glucose; amino acids; inorganic salts such as sodium selenite and sodium chloride; proteins such as transferrin; hormones such as insulin; cytokines such as transforming growth factor-β (TGF-β) and epidermal growth factor (EGF); growth factors; differentiation inhibitory factors; and antioxidants such as 2-mercaptoethanol and dithiothreitol. A medium is supplemented with the above-described components while culturing pluripotent stem cells in order to keep the concentration within a desired range over the entire culture period. It is preferable that a medium does not contain serum and a serum substitute from the viewpoint of suppressing contamination of pluripotent stem cells with an antigenic substance, an infection source, or the like. The pH of a medium is, for example, 7.0 to 8.0, preferably 7.3 to 7.4.

In the culture step in the present exemplary embodiment, it is preferable to replace a medium as appropriate. Media used for the series of culture steps may hot have the same composition. The culture may be continued while replacing a medium with a medium having a different composition as long as pluripotent stem cells can be maintained in an undifferentiated state.

It is desirable that a medium has a moderate viscosity as a medium for suspension culture in order to prevent movement of cell aggregations and intimate attachment between cell aggregations. Here, the moderate viscosity means viscosity to such a degree that adhesion of cell aggregations does not occur without hindering medium replacement.

Means for imparting viscosity to a medium is not particularly limited, but it can be carried out, for example, by adding a water-soluble polymer to a medium at an appropriate concentration. Any water-soluble polymer can be used as the water-soluble polymer as long as the water-soluble polymer can impart moderate viscosity to a medium and does not adversely affect cells (does not have cytotoxicity) within a concentration range in which viscosity can be imparted. Examples thereof include polysaccharides such as cellulose and agarose, esters of polysaccharides such as methyl cellulose, ethyl cellulose, hydroxyethyl cellulose, hydroxypropyl cellulose, hydroxyethyl methyl cellulose, hydroxypropyl methyl cellulose, hydroxyethyl ethyl cellulose, hydroxypropyl ethyl cellulose, ethyl hydroxyethyl cellulose, dihydroxypropyl cellulose, and hydroxyethyl hydroxypropyl cellulose, synthetic polymers such as polyacrylamide, polyethylene oxide, polyvinyl pyrrolidone, ethylene glycol-propylene glycol copolymer, polyethylene imine, polyvinyl methyl ether, polyvinyl alcohol, polyacrylic acid, and maleic acid copolymer, biopolymers such as collagen, gelatin, hyaluronic acid, dextran, alginic acid, carrageenan, and starch, or artificial polymers (for example, elastin-like peptide) imitating these. These water-soluble polymers may be used alone or as a mixture of several kinds of water-soluble polymers. Copolymers of these water-soluble polymers may also be used. The water-soluble polymers are preferably methyl cellulose, polyethylene glycol, polyvinyl pyrrolidone, and carboxymethyl cellulose, or a mixture thereof, and more preferably methyl cellulose.

For example, in a case where methyl cellulose is added to a medium in order to impart viscosity, the concentration of methyl cellulose is preferably higher than 0.25 w/v% and lower than 0.5 w/v%. The concentration of methyl cellulose is preferably 0.26 w/v% to 0.3 w/v% and particularly preferably 0.28 w/v%. By setting the concentration of methyl cellulose to be within the above-described ranges, it is possible to obtain a thickening action while maintaining permeability with respect to a mesh-like film used for division of cell aggregations in a dividing step and to locally block the mesh-like film, thereby suppressing variation in speed. The moderate viscosity thereof is represented by kinematic viscosity. For example, the viscosity at 25°C and at a shear rate of 100 $s^{-1}$ is 1.0 mPa·s to 15 mPa·s and more preferably 1.0 mPa·s to 7.5 mPa·s. Even in a case where other water-soluble polymers are used, those skilled in the art can appropriately select the concentration of other water-soluble polymers in order to obtain the above-described moderate viscosity. The viscosity at a shear rate of 100 $s^{-1}$ is evaluated using a viscosity measuring device (MCR301 with 25 mm cone plate and 0.098 mm gap, manufactured by Anton Paar GmbH) under the evaluation condition of interval setting 1.

Instead of imparting viscosity to a medium, it is also preferable to homogeneously form an amorphous structure in a medium for suspension culture and to suppress precipitation of cells without substantially increasing the viscosity of the medium. An example of a material capable of forming such a structure includes a polymer compound, and a preferred example thereof includes a polymer compound having an anionic functional group. Examples of the anionic functional group include a carboxy group, a sulfo group, a phosphoric acid group, and salts thereof, and a carboxy group or a salt thereof is preferable. The polymer compound is not particularly limited, but preferred specific examples thereof include polysaccharides obtained by polymerizing 10 or more monosaccharides (for example, triose, tetrose, pentose, hexose, and heptose), and more preferred examples thereof include acidic polysaccharides having anionic functional groups. More specific examples thereof include polymer compounds including one or more kinds from the group consisting of hyaluronic acid, gellan gum, deacylated gellan gum, rhamsan gum, diutan gum, xanthan gum, carrageenan, xanthan gum, hexuronic acid, fucoidan, pectin, pectic acid, pectinic acid, heparan sulfate, heparin, heparitin sulfate, keratosulfate, chondroitin sulfate, dermatan sulfate, rhamnan sulfate, and salts thereof. The polysaccharides are preferably hyaluronic acid, gellan gum, deacylated gellan gum, diutan gum, xanthan gum, carrageenan, or salts thereof, and it is possible to make cells and cell aggregations be suspended using polysaccharides at a low concentration. In addition, deacylated gellan gum is most preferable in consideration of easiness of collecting cells.

In a case where deacylated gellan gum is mixed with a liquid medium, the deacylated gellan gum takes in metal ions (for example, calcium ions) in the liquid medium to form an amorphous structure via metal ions and makes cells be suspended. The viscosity of the medium composition at a shear rate of 1000 $s^{-1}$ which is to be prepared by containing the deacylated gellan gum is less than or equal to 8 mPa·s, preferably less than or equal to 4 mPa·s, and more preferably 1.0 mPa·s to 2 mPa·s in consideration of easiness of collecting cells. The viscosity at a shear rate of 1000 $s^{-1}$ is evaluated using a viscosity measuring device (MCR301 with 25 mm cone plate and 0.098 mm gap, manufactured by Anton Paar GmbH) under the evaluation condition of interval setting 1.

Pluripotent stem cells subjected to adhesion culture are dissociated through enzymatic treatment and are seeded in a culture container so that the cell density becomes about $1 \times 10^5$ to $5 \times 10^6$ cells/ml and preferably about $2 \times 10^5$ to $2 \times 10^6$ cells/ml. The pluripotent stem cells are cultured in an atmosphere of a $CO_2$ concentration of about 1% to 10% and preferably about 2% to 5%, at a temperature of about 30°C to 40°C and preferably about 37°C, for 1 to 7 days, preferably 3 to 6 days, and more preferably 4 to 5 days. During the culture period, the medium in the culture container is preferably replaced with a fresh medium every 1 or 2 days.

In a case of culturing, for example, human ES cells which are divided once every about 24 hours, it is considered that the circle-equivalent diameters of cell aggregations of pluripotent stem cells which are set to about 80 $\mu$m at the start of suspension culture grow up to 200 $\mu$m to 300 $\mu$m through culture for 4 and 5 days.

[Subculture of Pluripotent Stem Cells]

Hereinafter, the dividing step according to the exemplary embodiment of the present disclosure which is to be performed in a case of performing subculture of pluripotent stem cells will be described. In the dividing step, the cell aggregations of pluripotent stem cells which have grown to have an average value of circle-equivalent diameters of 200 $\mu$m to 300 $\mu$m in the above-described culture step are divided into cell aggregations having smaller sizes.

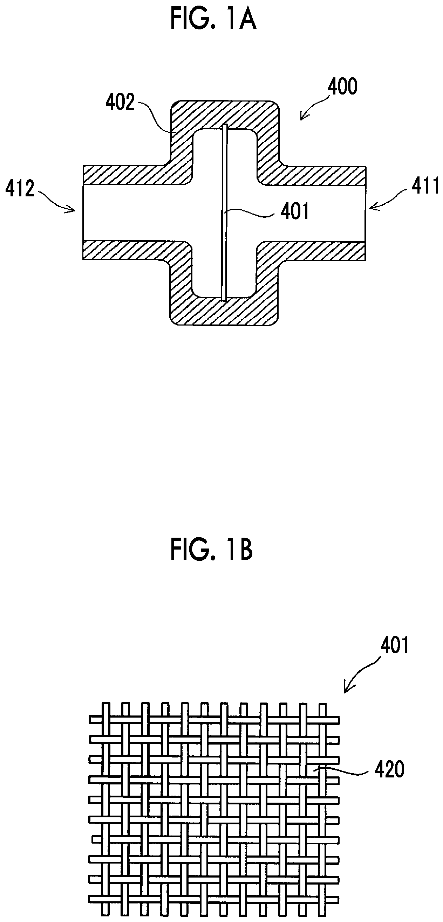

Fig. 1A is a cross-sectional view showing a configuration of a dividing device 400 used in the dividing step. The dividing device 400 includes a mesh 401 and a case 402.

The case 402 includes an inflow port 411 and an outflow port 412. The mesh 401 is provided between the inflow port 411 and the outflow port 412 in the case 402. That is, the inflow port 411 and the outflow port 412 are separated by the mesh 401.

Fig. 1B is a plan view showing a configuration of the mesh 401. The mesh 401 is formed by knitting fiber members, made of synthetic resin such as nylon or polyethylene terephthalate or metal such as stainless steel, in a lattice shape. That is, the mesh 401 is a mesh-like film having a plurality of through-holes 420. As shown in Fig. 1B, in a case where the warp and weft constituting the mesh 401 are knitted at the same intervals, the shapes of the through-holes 420 become square.

In the dividing step, the cell aggregations of pluripotent stem cells are made to flow into the dividing device 400 together with a medium from the inflow port 411 and made to flow out from the outflow port 412. When the cell aggregations of pluripotent stem cells flowing into the dividing device from the inflow port 411 pass through the mesh 401, the cell aggregations are divided into cell aggregations having smaller sizes. The speed at which the cell aggregations of pluripotent stem cells pass through the mesh 401 is set to 15 cm/sec to 150 cm/sec. By setting the speed at which the cell aggregations of pluripotent stem cells pass through the mesh to be greater than or equal to 401 to 15 cm/sec, it is possible to realize processing capacity applicable even to mass culture on a scale of, for example, 100 liters in the dividing step. In a case where the cross-sectional area of the flow path of the dividing device 400 in a direction orthogonal to a circulation direction of cell aggregations is set to 10 cm2, it is possible to set the amount of dividing processing per second to be greater than or equal to 150 cm$^3$. For example, even in a case where mass culture on a scale of 100 liters is performed, it is possible to complete the dividing processing in about 10 minutes and to maintain the homogeneity of the cells. In addition, by setting the speed at which cell aggregations of pluripotent stem cells pass through the mesh 401 to 15 cm/sec to 150 cm/sec, it is possible to suppress damage to the cells caused by division. The speed at which cell aggregations of pluripotent stem cells pass through the mesh 401 is more preferably 30 cm/sec to 120 cm/sec and still more preferably 50 cm/sec to 100 cm/sec from the viewpoints of the processing capacity and reduction in damage to cells.

The opening dimensions of the through-holes 420 of the mesh 401 are preferably 30 $\mu$m to 80 $\mu$m, more preferably 40 $\mu$m to 70 $\mu$m, and typically 50 $\mu$m. The opening dimension means a length of one side of a square in a case where the shapes of the through-holes 420 are square as shown in Fig. 1B, and means a diameter of a circle in a case where the shapes of the through-holes 420 are circular. By setting the opening dimensions of the through-holes 420 to be within the above-described ranges, it is possible to effectively divide cell aggregations while suppressing damage to the cell aggregations in a case where the cell aggregations pass through the mesh 401. Furthermore, it is possible to obtain a preferred distribution of the sizes of divided cell aggregations to be described below.

It is preferable that an average value of circle-equivalent diameters of divided cell aggregations becomes 30 $\mu$m to 75 $\mu$m. Here, the average value of circle-equivalent diameters of divided cell aggregations is measured as follows. For example, 300 cell aggregations divided by passing through the mesh 401 are randomly extracted under microscopic observation, the circle-equivalent diameter of each of the extracted cell aggregations is measured, and an average value of the measured circle-equivalent diameters is calculated. The measurement of the circle-equivalent diameters of the cell aggregations is preferably completed before 1 hour elapses after the division of the cell aggregations.

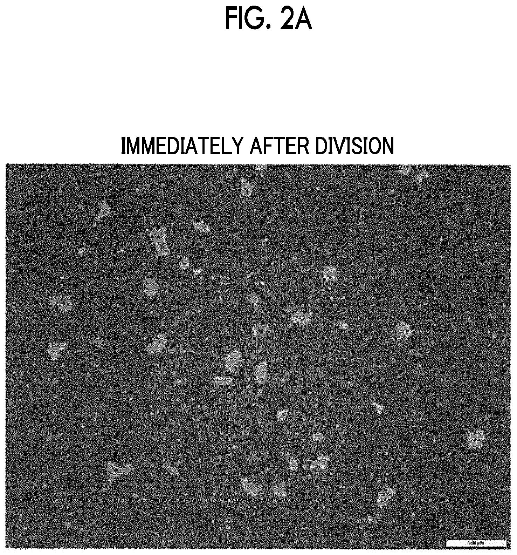

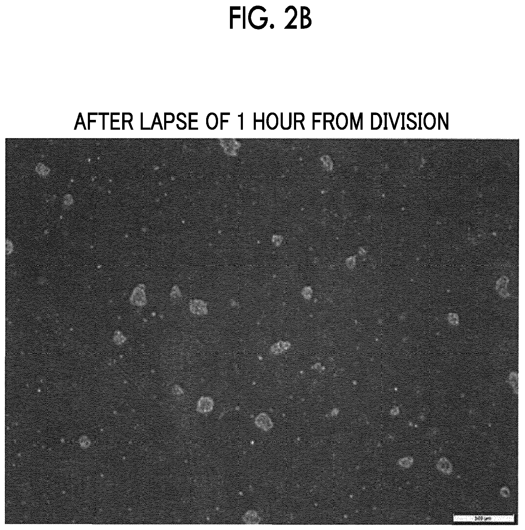

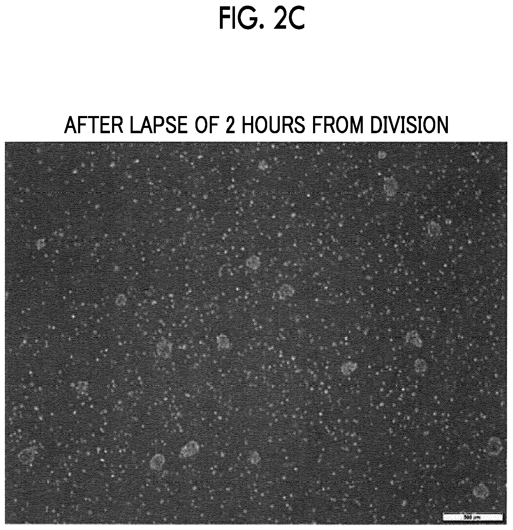

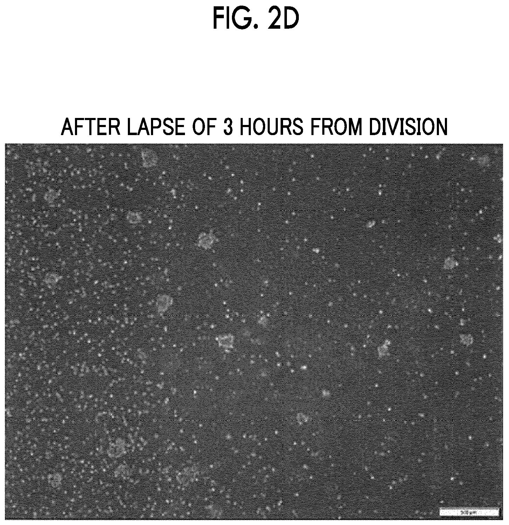

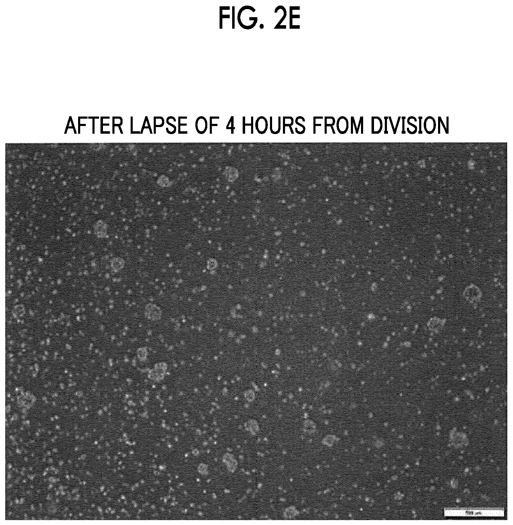

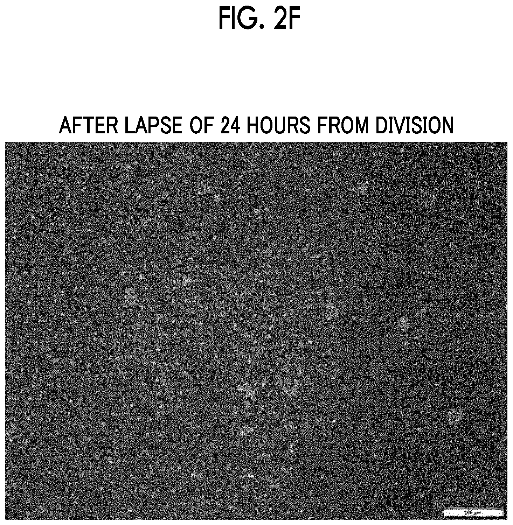

Figs. 2A to 2F are respectively micrographs showing states of cell aggregations immediately after division, after the lapse of 1 hour from the division, after the lapse of 2 hours from the division, after the lapse of 3 hours from the division, after the lapse of 4 hours from the division, and after the lapse of 24 hours from the division. As shown in Figs. 2A and 2B, the state of the cell aggregations in the period until 1 hour elapses after the division maintains the state of the cell aggregations immediately after division. On the other hand, in a case where 2 hours elapse after the division, a single cell as a dead cell is detached from the surfaces of the cell aggregations, and the number of single cells increases according to the lapse of time as shown in Figs. 2C to 2F. Accordingly, it is possible to obtain appropriate information as statistical information of cell aggregations immediately after the division, by completing the measurement of the sizes of the cell aggregations before 1 hour elapses after the division.

The number of cell aggregations having a circle-equivalent diameter of greater than or equal to 30 $\mu$m and less than 40 $\mu$m among the cell aggregations divided in the dividing step according to the present exemplary embodiment is set to X. In addition, the number of cell aggregations having a circle-equivalent diameter of greater than or equal to 40 $\mu$m and less than 300 $\mu$m among the cell aggregations divided in the dividing step according to the present exemplary embodiment is set to Y. In this case, it is preferable that the distribution of circle-equivalent diameters of the cell aggregations after the division satisfies Inequation (1).

$$1 < X / Y < 3 \cdots (1)$$

It is considered that damage to cell aggregations having circle-equivalent diameters of greater than or equal to 30 $\mu$m less than 40 $\mu$m caused by division is relatively large. However, it is considered that such cells to which damage is relatively large actively secrete proteins contributing to recovery from damage. It is considered that the proteins contribute not only to recovery of damage to own cells but also to recovery of damage to other cells. On the other hand, it is considered that damage to cell aggregations having circle-equivalent diameters of greater than or equal to 40 $\mu$m and less than 300 $\mu$m caused by division is relatively small and that the viability after division is relatively high. Accordingly, it is considered that, in a case where the distribution of circle-equivalent diameters of divided cell aggregations satisfies Inequation (1), it is possible to maximize the proliferation rate of cells after subculture (that is, after division).

By setting the opening dimensions of the through-holes 420 of the mesh 401 to 30 $\mu$m to 80 $\mu$m and setting the speed at which cell aggregations of the pluripotent stem cells pass through the mesh 401 to 15 cm/sec to 150 cm/sec, the average value of circle-equivalent diameters of the divided cell aggregations becomes 30 $\mu$m to 75 $\mu$m and it is easy to control the distribution of circle-equivalent diameters of the divided cell aggregations so as to satisfy Inequation (1). Accordingly, it is possible to efficiently divide cell aggregations while suppressing damage to pluripotent stem cells, and therefore, the method is suitable for mass culture of pluripotent stem cells.

Hereinafter, a cell culture device according to the exemplary embodiment of the present disclosure which can perform each treatment in the culture step and the dividing step according to the exemplary embodiment of the present disclosure described above, in a closed system will be described.

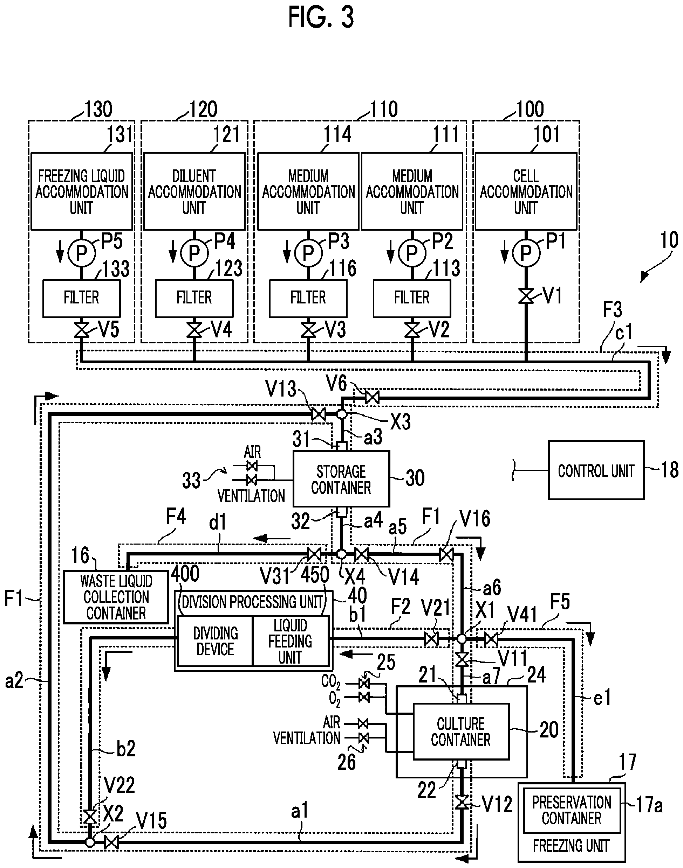

Fig. 3 is a diagram showing a configuration of a cell culture device 10 according to the exemplary embodiment of the present disclosure. The cell culture device 10 comprises a cell supply unit 100, a medium supply unit 110, a diluent supply unit 120, and a freezing liquid supply unit 130. In addition, the cell culture device 10 comprises a culture container 20, a storage container 30, a division processing unit 40, a waste liquid collection container 16, and a freezing unit 17.

The cell culture device 10 accommodates cells supplied from the cell supply unit 100 in the culture container 20 together with a medium (culture liquid) supplied from the medium supply unit 110, and the cells are cultured in the culture container 20 in a state of being suspended in the medium.

<Cell Supply Unit>

The cell supply unit 100 includes: a cell accommodation unit 101 that accommodates cells to be cultured by the cell culture device 10 in a frozen state; and a pump P1 which sends out the cells accommodated in the cell accommodation unit 101 to a flow path F3 configured to include a pipe c1. In addition, the cell supply unit 100 has an on-off valve V1 of a pipe connecting the cell accommodation unit 101 to the pipe c1, the on-off valve being provided on a downstream side of the pump P1. The cells accommodated in the cell accommodation unit 101 are sent out to the flow path F3 in a case where the pump P1 is driven and the on-off valve V1 enters an open state.

<Medium Supply Unit>

The medium supply unit 110 includes: medium accommodation units 111 and 114 each accommodating a medium (culture liquid) used for culturing cells; pumps P2 and P3 which send out the media respectively accommodated in the medium accommodation units 111 and 114 to the flow path F3; and filters 113 and 116 for sterilizing the media respectively sent out from the pumps P2 and P3. In addition, the medium supply unit 110 includes an on-off valve V2 of a pipe connecting the medium accommodation unit 111 to the pipe c1, the on-off valve being provided on a downstream side of the filter 113; and an on-off valve V3 of a pipe connecting the medium accommodation unit 114 to the pipe c1, the on-off valve being provided on a downstream side of the filter 116. In this manner, the medium supply unit 110 according to the present exemplary embodiment has a medium supply function in two systems consisting of a first system including the medium accommodation unit 111, the pump P2, the filter 113, and the on-off valve V2, and a second system including the medium accommodation unit 114, the pump P3, the filter 116, and the on-off valve V3, and it is possible to supply two different kinds of media. The number of systems in the medium supply unit 110 can be appropriately increased or decreased according to a cell culture protocol or the like. That is, the medium supply unit 110 may be configured to be able to supply three or more kinds of media, or may be configured to be able to supply one kind of medium. The medium accommodated in the medium accommodation unit 111 is sent out to the flow path F3 in a case where the pump P2 is driven and the on-off valve V2 enters an open state. The medium accommodated in the medium accommodation unit 114 is sent out to the flow path F3 in a case where the pump P3 is driven and the on-off valve V3 enters an open state.

<Diluent Supply Unit>

The diluent supply unit 120 includes: a diluent accommodation unit 121 which accommodates a diluent to be used for dilution processing which is to be appropriately performed during the process of culturing cells; a pump P4 which sends out the diluent accommodated in the diluent accommodation unit 121 to the flow path F3; and a filter 123 for sterilizing the diluent sent out from the pump P4. In addition, the diluent supply unit 120 has an on-off valve V4 of a pipe connecting the diluent accommodation unit 121 to the pipe c1, the on-off valve being provided on a downstream side of the filter 123. The diluent accommodated in the diluent accommodation unit 121 is sent out to the flow path F3 in a case where the pump P4 is driven and the on-off valve V4 enters an open state.

<Freezing Liquid Supply Unit>

The freezing liquid supply unit 130 includes: a freezing liquid accommodation unit 131 which accommodates a freezing liquid to be used in a case of freezing and preserving cultured cells in the freezing unit 17; a pump P5 which sends out the freezing liquid accommodated in the freezing liquid accommodation unit 131 to the flow path F3; and a filter 133 for sterilizing the freezing liquid sent out from the pump P5. In addition, the freezing liquid supply unit 130 includes an on-off valve V5 of a pipe connecting the freezing liquid accommodation unit 131, the pump P5, and the filter 133 to each other, the on-off valve being provided on a downstream side of the filter 133. The freezing liquid accommodated in the freezing liquid accommodation unit 131 is sent out to the flow path F3 in a case where the pump P5 is driven and the on-off valve V5 enters an open state. In a case where it is unnecessary to freeze and preserve cultured cells, it is possible to omit the freezing liquid supply unit 130 in the cell culture device 10.

<Culture Container>

The culture container 20 is a container for accommodating cells supplied from the cell supply unit 100 together with a medium supplied from the medium supply unit 110 and culturing the accommodated cells. The form of the culture container 20 is not particularly limited, and it is possible to use, for example, a container made of glass or stainless steel or a container having a form of a bag made of plastic. The culture container 20 includes: an inflow port 21 for allowing cells and a medium to flow into the culture container 20; and an outflow port 22 for allowing cells and a medium which are accommodated in the culture container 20 to flow out of the culture container 20.

The culture container 20 is accommodated in an incubator 24 which is sealed and of which the temperature and the $CO_2$ concentration are respectively controlled, for example, to 30°C to 40°C (preferably 37°C) and 2% to 10% (preferably 5%). The incubator 24 comprises a gas supply mechanism 25 for supplying oxygen ($O_2$) and carbon dioxide ($CO_2$) to cells accommodated in the culture container 20 together with the medium. In addition, the incubator 24 comprises a pressure adjustment mechanism 26 which adjusts the pressure in the culture container 20. The pressure adjustment mechanism 26 pressurizes the atmosphere in the culture container 20 by introducing air into the culture container 20, or releases the atmosphere in the culture container 20 to the atmospheric air by discharging the atmosphere in the culture container 20 to the outside. The pressure adjustment mechanism 26 allows cells and a medium which are accommodated in the culture container 20 to flow out into a circulation flow path F1 to be described below by increasing the pressure in the culture container 20 to be higher than that in the circulation flow path F1.

<Circulation Flow Path>

The cell culture device 10 includes the circulation flow path F1 configured to include pipes a1 to a7 which connect the outflow port 22 and the inflow port 21 of the culture container 20 to each other. The cells and the medium accommodated in the culture container 20 circulate in the circulation flow path F1 during the culture process. The cells and the medium flowing in the circulation flow path F1 flow into the culture container 20 via the inflow port 21 and the cells and the medium accommodated in the culture container 20 flow out into the circulation flow path F1 via the outflow port 22.

An on-off valve V11 is provided in a pipe a7 which constitutes the circulation flow path F1 and is connected to the inflow port 21 of the culture container 20, and an on-off valve V12 is provided in a pipe a1 which constitutes the circulation flow path F1 and is connected to the outflow port 22 of the culture container 20. The on-off valve V11 enters an open state in a case where cells and a medium are allowed to flow into the culture container 20 from the circulation flow path F1 and enters a closed state in other cases. The on-off valve V12 enters an open state in a case where cells and a medium are allowed to flow into the circulation flow path F1 from the inside of the culture container 20 and enters a closed state in other cases.

The flow path F3 constituted of the pipe c1 connected to the cell supply unit 100, the medium supply unit 110, the diluent supply unit 120, and the freezing liquid supply unit 130 is connected to the circulation flow path F1 at a connection site X3. That is, the cells accommodated in the cell accommodation unit 101, the media respectively accommodated in the medium accommodation units 111 and 114, the diluent accommodated in the diluent accommodation unit 121, and the freezing liquid accommodated in the freezing liquid accommodation unit 131 are supplied into the circulation flow path F1 via the flow path F3 and the connection site X3.

An on-off valve V6 is provided in the pipe c1 constituting the flow path F3 in the vicinity of the connection site X3. The on-off valve V6 enters an open state in a case where the cells, the media, the diluent and the freezing liquid are supplied into the circulation flow path F1 from the cell supply unit 100, the medium supply unit 110, the diluent supply unit 120, and the freezing liquid supply unit 130, respectively, and enters a closed state in other cases.

<Storage Container>

The storage container 30 is provided in the circulation flow path F1, that is, in the middle of the circulation flow path F1. The storage container 30 is a container for temporarily storing cells, a medium, a diluent, and a freezing liquid, which are to flow in the circulation flow path F1, and is used in culture start processing, medium replacement processing, dividing processing, and freezing processing which are to be described below and to be performed during the culture period. The form of the storage container 30 is not particularly limited, and it is possible to use, for example, a container made of glass or stainless steel or a container having a form of a bag made of plastic.

The storage container 30 includes: an inflow port 31 for allowing cells, a medium, a diluent, and a freezing liquid which are to flow in the circulation flow path F1 to flow into the storage container 30; and an outflow port 32 for allowing cells, a medium, a diluent, and a freezing liquid accommodated in the storage container 30 to flow out into the circulation flow path F1. The inflow port 31 of the storage container 30 is connected to the outflow port 22 of the culture container 20 by the pipes a1, a2, and a3 constituting the circulation flow path F1. The outflow port 32 of the storage container 30 is connected to the inflow port 21 of the culture container 20 by the pipes a4, a5, a6, and a7 constituting the circulation flow path F1. In addition, in the present exemplary embodiment, the connection site X3 at which the circulation flow path F1 is connected to the flow path F3 is disposed in the vicinity of the inflow port 31 of the storage container 30, but the position at which the circulation flow path F1 is connected to the flow path F3 can be disposed at any position in the circulation flow path F1.

An on-off valve V13 is provided in the pipe a2 constituting the circulation flow path F1 in the vicinity of the inflow port 31 of the storage container 30. The on-off valve V13 enters an open state in a case where cells, a medium, and the like are allowed to flow into the storage container 30 from the circulation flow path F1 and enters a closed state in other cases. An on-off valve V14 is provided in the pipe a5 constituting the circulation flow path F1 in the vicinity of the outflow port 32 of the storage container 30. The on-off valve V14 enters an open state in a case where cells, a medium, and the like are transferred from the inside of the storage container 30 the culture container 20, the division processing unit 40, and the freezing unit 17 through the inside of the circulation flow path F1, and enters a closed state in other cases.

In addition, the storage container 30 comprises a pressure adjustment mechanism 33 which adjusts the pressure in the storage container 30. The pressure adjustment mechanism 33 pressurizes the atmosphere in the storage container 30 by introducing air into the storage container 30, or releases the atmosphere in the storage container 30 to the atmospheric air by discharging the atmosphere in the storage container 30 to the outside. The pressure adjustment mechanism 33 allows cells, a medium, a diluent, and a freezing liquid which are stored in the storage container 30 to flow out into the circulation flow path F1 from the outflow port 32 by increasing the pressure in the storage container 30 to be higher than that in the circulation flow path F1.

The cell culture device 10 includes a flow path F2 configured to include pipes b1 and b2 which connect a connection site X1 positioned between the outflow port 32 of the storage container 30 and the inflow port 21 of the culture container 20 in the circulation flow path F1 to a connection site X2 positioned between the inflow port 31 of the storage container 30 and the outflow port 22 of the culture container 20 in the circulation flow path F1. Cells, a medium, and the like flowing in the circulation flow path F1 can flow into the flow path F2 via the connection site X1. In addition, cells, a medium, and the like flowing in the flow path F2 can flow into the circulation flow path F1 via the connection site X2.

<Division Processing Unit>

The division processing unit 40 is provided in the flow path F2, that is, in the middle of the flow path F2. The division processing unit 40 comprises the above-described dividing device 400 shown in Fig. 1A in the culture container 20. The division processing unit 40 divides cell aggregations flowing into the flow path F2 from the circulation flow path F1 via the connection site X1 in the dividing device 400. The cells subjected to the dividing processing flow out into the circulation flow path F1 via the connection site X2.

The division processing unit 40 comprises a liquid feeding unit 450 connected to the inflow port 411 (see Fig. 1A) of the dividing device 400. The liquid feeding unit 450 includes a liquid feeding mechanism for sending out a medium containing cell aggregations to the dividing device 400. The liquid feeding unit 450 may have a form of a so-called syringe pump which pushes a liquid accommodated in a cylindrical container using a piston. The speed at which cell aggregations of pluripotent stem cells pass through the mesh 401 of the dividing device 400 is controlled by the liquid feeding unit 450. The liquid feeding unit 450 performs liquid feeding at a constant speed within a range of 15 cm/sec to 150 cm/sec at which cell aggregations of pluripotent stem cells pass through the mesh 401 of the dividing device 400.

An on-off valve V21 is provided in the pipe b1 which constitutes the flow path F2 and is disposed on an upstream side of the division processing unit 40 in the vicinity of the connection site X1. The on-off valve V21 enters an open state in a case where cells and the like are transferred to the division processing unit 40 from the storage container 30 and enters a closed state in other cases. On the other hand, an on-off valve V22 is provided in the pipe b2 which constitutes the flow path F2 and is disposed on a downstream side of the division processing unit 40 in the vicinity of the connection site X2. The on-off valve V22 enters an open state in a case where cells, subjected to dividing processing by the division processing unit 40, are allowed to flow out into the circulation flow path F1 and enters a closed state in other cases.

An on-off valve V15 is provided in the pipe a1 constituting the circulation flow path F1 on an upstream side of the connection site X2 in the vicinity of the connection site X2. The on-off valve V15 enters an open state in a case where cells, a medium, and the like are transferred to the storage container 30 from the culture container 20 and enters a closed state in other cases.

<Waste Liquid Collection Container>

The cell culture device 10 includes a flow path F4 configured to include a pipe d1 connected to the circulation flow path F1 at a connection site X4 positioned on an upstream side of the connection site X1 (near the storage container 30), between the outflow port 32 of the storage container 30 and the inflow port 21 of the culture container 20 in the circulation flow path F1. The waste liquid collection container 16 is provided at the end of the flow path F4. The waste liquid collection container 16 is a container for collecting a waste liquid flowing into the flow path F4 from the circulation flow path F1 via the connection site X4. A waste liquid collected in the waste liquid collection container 16 contains a used medium, a used diluent, a freezing liquid, and the like accompanied by cells supplied from the cell supply unit 100 in a frozen state. The form of the waste liquid collection container 16 is not particularly limited, and it is possible to use, for example, a container made of glass or stainless steel or a container having a form of a bag made of plastic.

An on-off valve V31 is provided in the pipe d1 constituting the flow path F4 in the vicinity of the connection site X4. The on-off valve V31 enters an open state in a case where a waste liquid flowing out from the storage container 30 is collected in the waste liquid collection container 16 and enters a closed state in other cases.

<Freezing Unit>

The cell culture device 10 includes a flow path F5 configured to include a pipe e1 connected to the circulation flow path F1 at the connection site X1. The freezing unit 17 is provided at the end of the flow path F5. The freezing unit 17 includes a preservation container 17a for accommodating cells flowing into the flow path F5 from the circulation flow path F1 via the connection site X1 together with a freezing liquid supplied from the freezing liquid supply unit 130. The preservation container 17a may have a form of, for example, a vial, a cryotube, or a bag. The freezing unit 17 can be configured to include a freezer which freezes a freezing liquid and cells accommodated in the preservation container 17a. In addition, the freezing unit 17 may comprise a tank filled with liquid nitrogen, or may be configured to be able to accommodate the preservation container 17a in the tank. In addition, the freezing unit 17 may be configured to include, for example, a CRYOLIBRARY (registered trademark) system manufactured by TAIYO NIPPON SANSO CORPORATION. An on-off valve V41 is provided in the pipe e1 constituting the flow path F5 in the vicinity of the connection site X1. The on-off valve V41 enters an open state in a case where cells and a freezing liquid are transferred to the freezing unit 17 from the storage container 30 and enters a closed state in other cases. The position at which the flow path F5 is connected to the circulation flow path F1 may be any position as long as it is between the outflow port 32 of the storage container 30 and the inflow port 21 of the culture container 20. In addition, in a case where it is unnecessary to freeze and preserve cells, it is possible to omit the freezing unit 17.

<Control Unit>

The control unit 18 integrally controls operations of the pumps P1 to P5, on-off valves V1 to V5, V11 to V16, V21, V22, V31, and V41, the gas supply mechanism 25, and the pressure adjustment mechanisms 26, 33, and 43. Accordingly, culture of cells in accordance with a predetermined cell culture protocol is automatically performed without human intervention. In Fig. 3, an illustration of electrical connection wiring between the control unit 18 and each of the above-described constituent elements controlled by the control unit 18 is omitted from the viewpoint of avoiding complication of the drawing.

An example of processing that can be carried out in the cell culture device 10 according to the present exemplary embodiment will be described below. The cell culture device 10 performs, for example, culture start processing, medium replacement processing, dividing processing, and freezing processing to be exemplified below. The culture start processing, medium replacement processing, dividing processing, and freezing processing to be described below are performed by the control unit 18 which controls operations of the on-off valve V1 to V5, V11 to V16, V21, V22, V31, and V41, the pumps P1 to P5, and the pressure adjustment mechanisms 26, 33, and 43.

<Culture Start Processing>

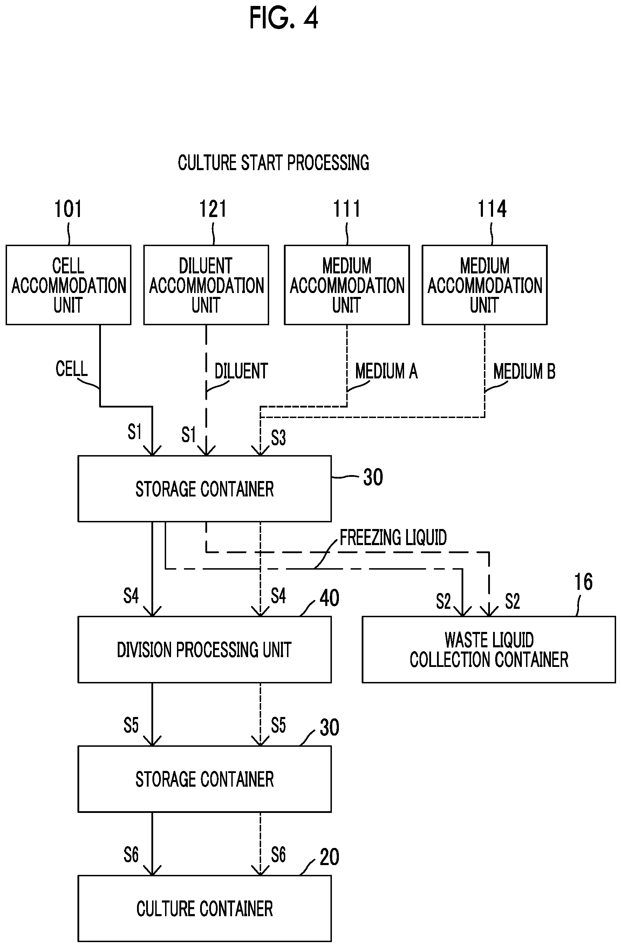

The cell culture device 10 performs culture start processing of starting cell culture by accommodating cells accommodated in the cell accommodation unit 101 in the culture container 20 together with media accommodated in the medium accommodation units 111 and 114 as follows. Fig. 4 is a diagram showing a flow of cells, a medium, and the like in a case where the cell culture device 10 performs the culture start processing. The correspondence between the flow of cells, a medium, and the like and the processing steps shown below is shown in Fig. 4.

In step S1, the on-off valves V1, V4, and V6 enter an open state, and the pumps P1 and P4 are driven. Accordingly, cells accommodated in the cell accommodation unit 101 in a frozen state and a diluent accommodated in the diluent accommodation unit 121 flow into the storage container 30 via the flow path F3 and the circulation flow path F1.

In step S2, concentration processing for removing a freezing liquid and the diluent from a mixture containing the cells stored in the storage container 30 and the diluent and the freezing liquid accompanied by the cells is performed. The above-described concentration processing is carried out, for example, by precipitating (naturally precipitating) the cells, which are accommodated together with the diluent in the storage container 30, in the storage container 30 and removing a supernatant containing the diluent and the freezing liquid. Specifically, the on-off valve V14 briefly enters an open state after the cells are precipitated in the storage container 30. Accordingly, the cells are made to flow out from the storage container 30 while leaving the supernatant in the storage container 30 and are allowed to stay in the pipe a5. Thereafter, the on-off valve V14 enters a closed state and the on-off valve V31 enters an open state. Accordingly, the waste liquid, which contains the diluent and the freezing liquid and remains in the storage container 30, flows out from the outflow port 32 of the storage container 30, and is collected in the waste liquid collection container 16 via the flow path F4. The transfer of the cells from the storage container 30 to the pipe a5 and the transfer of the diluent and the freezing liquid from the storage container 30 to the waste liquid collection container 16 are performed by pressurizing the atmosphere in the storage container 30 using the pressure adjustment mechanism 33.

In step S3, the on-off valves V2, V3, and V6 enter an open state, and the pumps P2 and P3 are driven. Accordingly, media A and B accommodated in the medium accommodation units 111 and 114 flow into the storage container 30 via the flow path F3 and the circulation flow path F1. Thereafter, the on-off valve V14 enters an open state, and the mixed medium containing the media A and B flows out from the storage container 30 and joins the cells staying in the pipe a5.

In step S4, the on-off valves V14, V16, and V21 enter an open state. In addition, the atmosphere in the storage container 30 is pressurized by the pressure adjustment mechanism 33. Accordingly, the cells and the medium staying in the pipe a5 flow into the flow path F2 via the connection site X1 and flow into the division processing unit 40. The cells flowing into the division processing unit 40 are subjected to dividing processing in the dividing device 400. The dividing processing here is carried out for the purpose of crushing the frozen cells.

In step S5, the on-off valves V22 and V13 enter an open state, and the cells subjected to the dividing processing flow out into the circulation flow path F1 via the connection site X2 together with the medium, and are transferred into the storage container 30.

The on-off valves V14, V16, and V11 enter an open state in step S6. In addition, the atmosphere in the storage container 30 is pressurized by the pressure adjustment mechanism 33. Accordingly, the cells and the medium which are stored in the storage container 30 flow into the culture container 20 via the circulation flow path F1. The culture start processing is completed through the above-described processing of steps S1 to S6. In the above-described example, the concentration processing and supply of a fresh medium are carried out before the dividing processing, but the concentration processing and the supply of a fresh medium may be carried out after the dividing processing.

<Medium Replacement Processing>

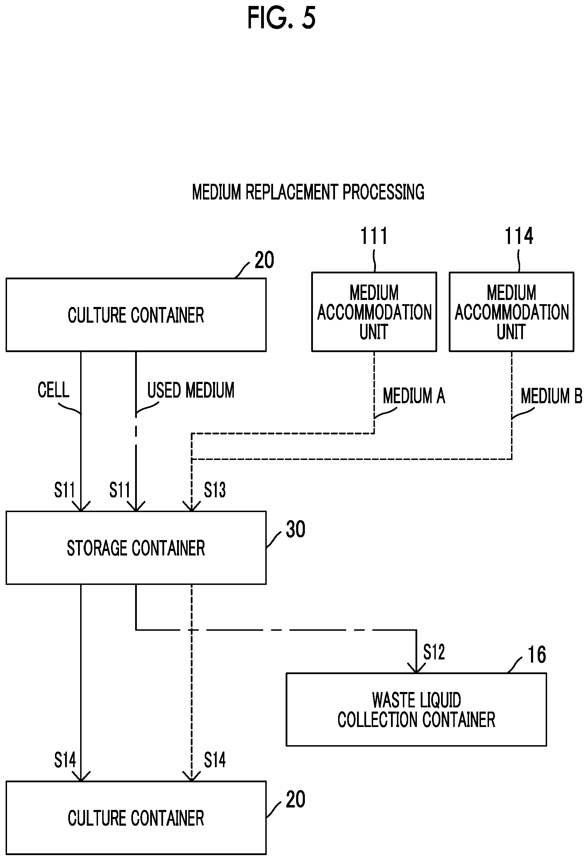

In culturing of cells, the medium is altered by metabolites or the like secreted from the cells. For this reason, medium replacement processing becomes necessary which replaces a used medium in the culture container 20 with a fresh medium at appropriate time within a culture period. In the cell culture device 10 according to the present exemplary embodiment, the above-described medium replacement processing is performed as follows. Fig. 5 is a diagram showing a flow of cells, a medium, and the like in a case where the cell culture device 10 performs the medium replacement processing. The correspondence between the flow of cells, a medium, and the like and the processing steps shown below is shown in Fig. 5.

The on-off valves V12, V15, and V13 enter an open state in step S11. In addition, the atmosphere in the culture container 20 is pressurized by the pressure adjustment mechanism 26. Accordingly, the cells and the used medium which are accommodated in the culture container 20 flow out into the circulation flow path F1 and are transferred into the storage container 30.

Concentration processing for removing the used medium from the mixture containing the cells and the used medium which are stored in the storage container 30 is performed in step S12. The concentration processing is performed in the same procedure as the processing in step S2 of the culture start processing described above. The used medium is collected in the waste liquid collection container 16 through the concentration processing and the cells stay in the pipe a5. A diluent may be introduced into the storage container 30 in order to promote precipitation of the cells.

The on-off valves V2, V3, and V6 enter an open state, and the pumps P2 and P3 are driven in step S13. Accordingly, fresh media A and B accommodated in the medium accommodation units 111 and 114 flow into the storage container 30 via the flow path F3 and the circulation flow path F1. Thereafter, the on-off valve V14 enters an open state, and the fresh medium containing the media A and B flows out from the storage container 30 and joins the cells staying in the pipe a5.

The on-off valves V14, V16, and V11 enter an open state in step S14. In addition, the atmosphere in the storage container 30 is pressurized by the pressure adjustment mechanism 33. Accordingly, the cells and the fresh medium which stay in the pipe a5 flow into the culture container 20. The cells accommodated in the culture container 20 are cultured using the fresh medium. The medium replacement processing is completed through the above-described processing of steps S11 to S 14.

<Dividing Processing>

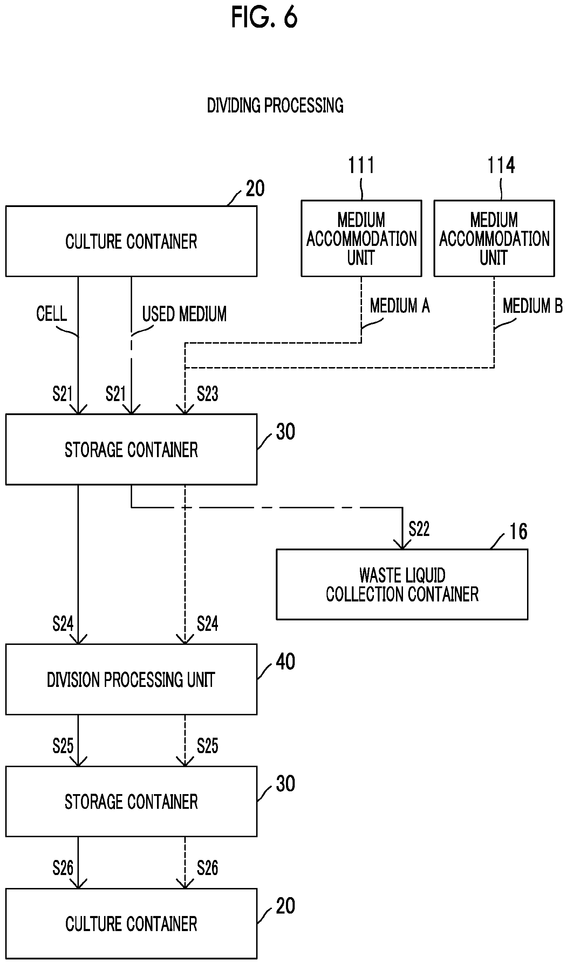

In culturing of pluripotent stem cells, in a case where sizes of cell aggregations (spheres) generated by culturing cells become too large, problems can occur; for example, the cell aggregations adhere to and are fused with each other, cells start to differentiate, or cells in central portions of the cell aggregations are necrotized. Accordingly, in order to prevent the sizes of cell aggregations from becoming too large, in some cases, dividing processing for dividing the cell aggregations is necessary at an appropriate time during the culture period. In the cell culture device 10 according to the present exemplary embodiment, the above-described dividing processing is performed as follows. Fig. 6 is a diagram showing a flow of cells, a medium, and the like in a case where the cell culture device 10 performs the dividing processing. The correspondence between the flow of cells, a medium, and the like and the processing steps shown below is shown in Fig. 6.

The on-off valves V12, V15, and V13 enter an open state in step S21. In addition, the atmosphere in the culture container 20 is pressurized by the pressure adjustment mechanism 26, and the cell aggregations and the used medium generated in the culture container 20 flow out into the circulation flow path F1 and are transferred into the storage container 30.

Concentration processing for removing the used medium from the mixture containing the cell aggregations and the used medium which are stored in the storage container 30 is performed in step S22. The concentration processing is performed in the same procedure as the processing in step S2 of the culture start processing described above. The used medium is collected in the waste liquid collection container 16 through the concentration processing and the cell aggregations stay in the pipe a5. A diluent may be introduced into the storage container 30 in order to promote precipitation of the cells.

The on-off valves V2, V3, and V6 enter an open state, and the pumps P2 and P3 are driven in step S23. Accordingly, fresh media A and B accommodated in the medium accommodation units 111 and 114 flow into the storage container 30 via the flow path F3 and the circulation flow path F1. Thereafter, the on-off valve V14 enters an open state, and the fresh medium containing the media A and B flows out from the storage container 30 and joins the cell aggregations staying in the pipe a5.

The on-off valves V14, V16, and V21 enter an open state in step S24. In addition, the atmosphere in the storage container 30 is pressurized by the pressure adjustment mechanism 33. Accordingly, the cell aggregations and the medium staying in the pipe a5 flow into the flow path F2 via the connection site X1 and flow into the division processing unit 40. The cells flowing into the division processing unit 40 are divided by the dividing device 400. The speed at which cell aggregations of pluripotent stem cells pass through the mesh 401 of the dividing device 400 is controlled by the liquid feeding unit 450. The liquid feeding unit 450 performs liquid feeding at a constant speed within a range of 15 cm/sec to 150 cm/sec at which cell aggregations of pluripotent stem cells pass through the mesh 401 of the dividing device 400.

In step S25, the on-off valves V22 and V13 enter an open state, and the cells subjected to the dividing processing flow out into the circulation flow path F1 via the connection site X2 together with the medium, and are transferred into the storage container 30.

The on-off valves V14, V16, and V11 enter an open state in step S26. In addition, the atmosphere in the storage container 30 is pressurized by the pressure adjustment mechanism 33. Accordingly, the cells and the fresh medium which are stored in the storage container 30 and subjected to dividing processing flow into the culture container 20. In the culture container 20, culturing of the cells subjected to the dividing processing is continued. The dividing processing is completed through the above-described processing of steps S21 to S26. That is, the subculture of the cells is completed.

In the above-described example, the concentration processing and supply of a fresh medium are carried out before the dividing processing, but the concentration processing and the supply of a fresh medium may be carried out after the dividing processing.

<Freezing Processing>

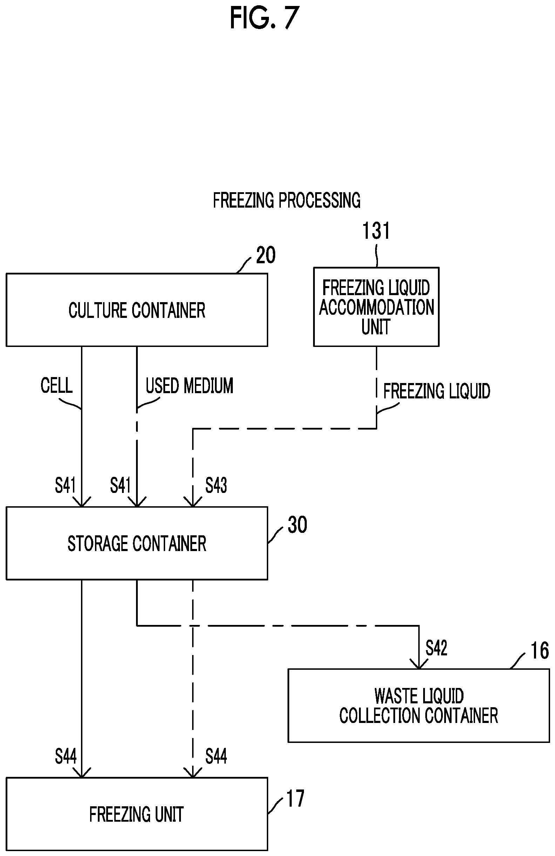

In a case of collecting and storing cultured cells, it is common that the cells are collected in a preservation container to be frozen and preserved. The cell culture device 10 according to the present exemplary embodiment performs the freezing processing in which cultured cells are collected and frozen as follows. Fig. 7 is a diagram showing a flow of cells, a medium, and the like in a case where the cell culture device 10 performs the freezing processing. The correspondence between the flow of cells, a medium, and the like and the processing steps shown below is shown in Fig. 7.

The on-off valves V12, V15, and V13 enter an open state in step S41. In addition, the atmosphere in the culture container 20 is pressurized by the pressure adjustment mechanism 26. Accordingly, the cells and the used medium which are accommodated in the culture container 20 flow out into the circulation flow path F1 and are transferred into the storage container 30.

Concentration processing for removing the used medium from the mixture containing the cells and the used medium which are stored in the storage container 30 is performed in step S42. The concentration processing is performed in the same procedure as the processing in step S2 of the culture start processing described above. The used medium is collected in the waste liquid collection container 16 through the concentration processing and the cells stay in the pipe a5. A diluent may be introduced into the storage container 30 in order to promote precipitation of the cells.

The on-off valves V5 and V6 enter an open state, and the pump P5 is driven in step S43. Accordingly, a freezing liquid accommodated in the freezing liquid accommodation unit 131 flows into the storage container 30 via the flow path F3 and the circulation flow path F1. Thereafter, the on-off valve V14 enters an open state, and the freezing liquid flows out from the storage container 30 and joins the cells staying in the pipe a5.

The on-off valves V14, V16, and V41 enter an open state in step S44. In addition, the atmosphere in the storage container 30 is pressurized by the pressure adjustment mechanism 33. Accordingly, the cells and the freezing liquid which stay in the pipe a5 are accommodated in the preservation container 17a of the freezing unit 17 via the flow path F5. The freezing unit 17 freezes the cells accommodated in the preservation container 17a together with the freezing liquid. The freezing processing is completed through the above-described processing of steps S41 to S44.

A method disclosed in JP4705473B may be applied as a method for freezing and preserving cells, for example. This method includes a step of rapidly freezing cells in a medium containing a predetermined amount of dimethyl sulfoxide (DMSO), propylene glycol, acetamide, and a medium. In addition, the method described in JP4223961B may be applied. This method is a method for freezing and preserving cells in which a cryopreservation liquid containing at least one selected from the group consisting of dimethyl sulfoxide, glycerin, ethylene glycol, propylene glycol, and polyvinyl pyrrolidone at a predetermined concentration as a cryoprotective agent is used, the method including: a step of suspending cells in the cryopreservation liquid, a cooling step of cooling and freezing the cryopreservation liquid to a temperature of lower than or equal to -80°C at a predetermined cooling rate, and a preserving step of preserving the cryopreservation liquid at a temperature of lower than or equal to -80°C.

A case where two types of media A and B are supplied from the medium supply unit 110 in the culture start processing, the medium replacement processing, and the dividing processing described above, but the type of medium to be used can be appropriately changed according to a cell culture protocol. That is, the medium to be used may be one type or three or more types depending on the culture protocol.

<Cell Culture Processing>

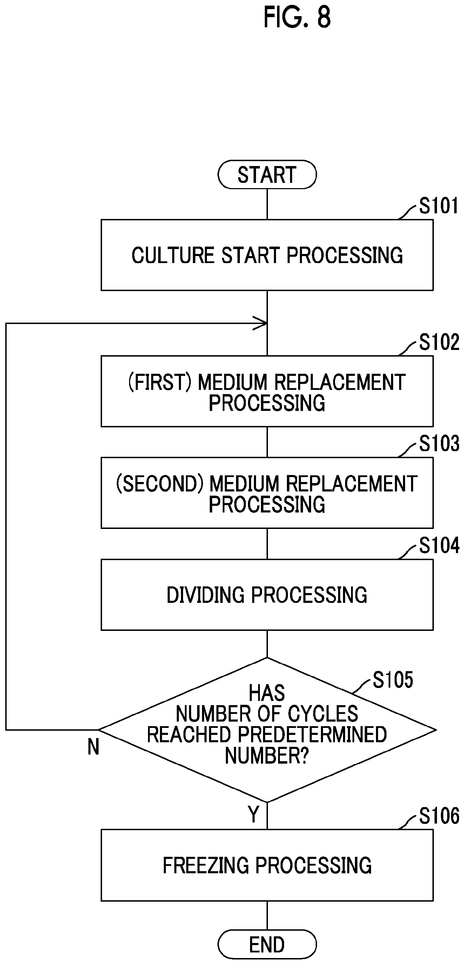

The cell culture device 10 can automatically perform cell culture using the control unit 18 executing the cell culture processing program to be exemplified below without human intervention. Fig. 8 is a flowchart showing a flow of processing in a cell culture program executed by the control unit 18.

In step S101, cells supplied from the cell supply unit 100 and media supplied from the medium supply unit 110 are accommodated in the culture container 20 and cell culture starts using the control unit 18 by performing the above-described culture start processing.

In step S102, the used media in the culture container 20 are replaced with fresh media accommodated in the medium accommodation units 111 and 114 and the cell culture is continued by the control unit 18 performing the above-described (first) medium replacement processing after a predetermined period of time elapses from the start of the cell culture.

In step S103, the used media in the culture container 20 are replaced with fresh media accommodated in the medium accommodation units 111 and 114 and the cell culture is continued by the control unit 18 performing the above-described (second) medium replacement processing after a predetermined period of time elapses from the execution of the first medium replacement processing.

In step S104, the control unit 18 divides cell aggregations and the cell culture is continued by performing the above-described dividing processing after a predetermined period of time elapses from the execution of the second medium replacement processing.

In step S105, the control unit 18 determines whether or not the number of culture cycles in which the above-described processing of steps S102 to S104 is set to one cycle has reached a predetermined number. In a case where the control unit 18 determines that the number of culture cycles has not reached a predetermined number, the control unit 18 returns the processing back to step S102. In a case where the control unit 18 determines that the number of culture cycles has reached a predetermined number of cycles, the control unit 18 advances the processing to step S106. As the culture cycle progresses, the scale of culturing cells increases.

In step S106, the cultured cells are accommodated in the preservation container 17a of the freezing unit 17 and frozen and preserved by the control unit 18 performing the above-described freezing processing.

In the above-described example, the medium replacement processing is performed twice within one culture cycle, but the number of times of performing the medium replacement processing within one culture cycle can be appropriately changed.

As described above, the cell culture device 10 according to the present exemplary embodiment includes the circulation flow path F1 connecting the outflow port 22 and the inflow port 21 of the culture container 20 to each other. In addition, the cell culture device 10 includes the storage container 30 provided in the circulation flow path F1. The storage container 30 includes the inflow port 31 connected to the outflow port 22 of the culture container 20 and the outflow port 32 connected to the inflow port 21 of the culture container 20. The storage container 30 is provided for concentration processing or the like performed in the above-described culture start processing, medium replacement processing, dividing processing, and freezing processing. In addition, the cell culture device 10 includes the flow path F2 connecting the connection site X1 positioned between the outflow port 32 of the storage container 30 and the inflow port 21 of the culture container 20 in the circulation flow path F1 to the connection site X2 positioned between the inflow port 31 of the storage container 30 and the outflow port 22 of the culture container 20 in the circulation flow path F1. The cell culture device 10 includes the division processing unit 40 which is provided in the flow path F2, divides a cell aggregation flowing in from the circulation flow path F1 via the connection site X1, and allows the divided cell aggregations to flow out into the circulation flow path F1 via the connection site X2. In addition, the cell culture device 10 includes the medium supply unit 110 which supplies a medium to the inside of the circulation flow path F1.

By configuring the cell culture device 10 as described above, it is possible to continuously perform a series of processings, such as medium replacement processing and dividing processing, which are required for cell culture, in a closed system. Accordingly, this enables mass production of homogeneous cells. According to the cell culture device 10 of the present exemplary embodiment, it is possible to perform a series of processings from the culture start processing to the freezing processing required for cell culture without manual intervention.

A case where the process from the culture start processing to the freezing processing is automated by the control unit 18 controlling operations of the on-off valves V1 to V5, V11 to V16, V21, V22, V31, and V41, the pumps P1 to P5, and the pressure adjustment mechanisms 26, 33, and 43 is exemplified in the cell culture device 10 according to the present exemplary embodiment, but this aspect is not limited thereto. A user may manually operate the on-off valves V1 to V5, V11 to V16, V21, V22, V31, and V41, the pumps P1 to P5, and the pressure adjustment mechanisms 26, 33, and 43.

Example

Hereinafter, the exemplary embodiment of the invention will be described in detail using an example, but the exemplary embodiment of the invention is not limited to the example.

In the following, "M" represents a molar concentration relating to the substance concentration, and 1 M is 1 mol/L.

In the following, "PBS" means phosphate buffered saline and "IMDM" means an Iscove's modified Dulbecco's medium.

In the following, the "sphere" means a spherical cell aggregation.

<Materials>

[Human Induced Pluripotent Stem Cell Line (hiPS Cell Line)]

• 253G1, Distributed from iPS PORTAL, Inc. (448-5 Kajii-cho, Imadegawa-Sagaru, Kawaramachi-dori, Kamigyo-ku, Kyoto, Japan)

[Basic Medium and Medium Additive]

- TeSR-E8, model number ST-05940 of STEMCELL Technologies
- 3% methyl cellulose solution (in IMDM, methyl cellulose concentration is w/v%), model number HSC001 from R&D Systems
- 10 mM Y-27632 solution which is solution obtained by dissolving Y-27632 (ROCK inhibitor, model number Y0503 of Sigma-Aldrich) in Dulbecco's PBS (Ca- and Mg-free)

[Medium]

- A medium 1 was prepared such that 50 mL of a 3% methyl cellulose solution was added to 450 mL of TeSR-E8 and the mixture was thoroughly stirred. A 10 mM Y-27632 solution was added thereto in such an amount that the final concentration of Y-27632 became 10 $\mu$M.
- A medium 2 was prepared such that 50 mL of a 3% methyl cellulose solution was added to 450 mL of TeSR-E8, and the mixture was thoroughly stirred.

[Culture Dish and Centrifuge Tube]

- Ultra-Low Attachment Plate with 6 wells and lid, model number Costar 3471 of Corning
- 15 mL Centrifuge tube, model number 339650 of Thermo Fisher Scientific Inc.
- 50 mL Centrifuge tube, model number 339652 of Thermo Fisher Scientific Inc.

[Mesh for Subculture]

- Mesh (1): Partec CellTrics with model number 06-04-004-2325 of Sysmex Corporation
- Mesh (2): Partec CellTrics with model number 06-04-004-2326 of Sysmex Corporation
- Mesh (3): Cell strainer with model number 352340 of Becton, Dickinson and Company
- Mesh (4): Partec CellTrics with model number 06-04-004-2327 of Sysmex Corporation
- Mesh (5): Cell strainer with model number 352350 of Becton, Dickinson and Company
- Mesh (6): Cell strainer with model number 352360 of Becton, Dickinson and Company

The meshes (1) to (6) were observed with a phase contrast microscope (IX73 of Olympus Corporation) at a magnification of 20 times, and the opening dimensions of the meshes were obtained from an average obtained by measuring two sides of the opening. The opening dimensions of the meshes were as follows.

- Mesh (1): Opening dimension of 25.7 $\mu$m
- Mesh (2): Opening dimension of 35.9 $\mu$m
- Mesh (3): Opening dimension of 40.5 $\mu$m
- Mesh (4): Opening dimension of 43.3 $\mu$m
- Mesh (5): Opening dimension of 73.1 $\mu$m
- Mesh (6): Opening dimension of 100.1 $\mu$m

All the meshes for subculture were sterilized using an autoclave and used for a subculture operation.

<Example: Culture and Subculture of hiPS Cells>

[Culture of hiPS Cells]

A hiPS cell line 253G1 was subjected to suspension culture for 5 days while replacing a medium in an incubator under the conditions of 37°C and 5% $CO_2$ using a 6-well culture dish and a medium 2 to form spheres.

[Subculture of hiPS Cells]

After suspension culture for 5 days, the 6-well culture dish was taken out from the incubator, and the spheres were gathered in the centers of the wells by moving the wells so as to draw a circle and were transferred to a 15 mL centrifuge tube together with the medium. 3 mL of TeSR-E8 previously heated to 37°C was placed in the wells, and the spheres remaining in the wells were gathered and further transferred to the 15 mL centrifuge tube.

After removing a supernatant by subjecting the 15 mL centrifuge tube to centrifugal processing (50 rpm, 2 minutes), a medium 1 previously heated to 37°C was placed to re-suspend the spheres in the medium 1. The sphere suspension was transferred to another 15 mL centrifuge tube containing the medium 1 (liquid temperature of 37°C) to prepare a sphere floating liquid having a cell concentration of $5 \times 10^5$/mL.

A mouth of a syringe (model number SS50-LZ of TERUMO CORPORATION) and one end of a silicon tube were connected to each other via a luer lock type tube connector, and the other end of the silicon tube and a pipette tip (model number 607160 of Greiner Bio-One with distal end inner diameter 1.5 mm) were connected to each other via the tube connector. The sphere floating liquid was transferred to the syringe, and the syringe was loaded in a syringe pump (model number PHD-2000 of Harvard).

Each of the meshes (1) to (6) was attached to a mouth of a 15 mL centrifuge tube or a 50 mL centrifuge tube. A distal end of a pipette tip connected to the syringe accommodating the sphere floating liquid was pressed against the mesh, the syringe pump was operated to extrude the sphere floating liquid, and the sphere floating liquid was passed through the mesh to divide the spheres. The speed at which the sphere floating liquid passed through the mesh was varied by controlling the flow rate with the syringe pump.

[Culture After Subculture]

The sphere floating liquid after being passed through the mesh was seeded in a 6-well culture dish and cultured in an incubator under the conditions of 37°C and 5% $CO_2$.

On day 1 of the culture after the subculture (that is, 24 hours after the passage through the mesh), the 6-well culture dish was taken out from the incubator, and the spheres were gathered in the centers of the wells by moving the wells so as to draw a circle and were transferred to a 15 mL centrifuge tube together. 3 mL of TeSR-E8 previously heated to 37°C was placed in the wells, and the spheres remaining in the wells were gathered and further transferred to the 15 mL centrifuge tube.

After removing a supernatant by subjecting the 15 mL centrifuge tube to centrifugal processing (50 rpm, 2 minutes), a medium 2 previously heated to 37°C was placed in an amount equal to that of a medium before replacement to re-suspend the spheres, and the medium replacement was performed. The sphere suspension was returned to the wells, and the culture was continued in the incubator under the conditions of 37°C and 5% $CO_2$.

The same operation as above was also carried out on day 3 of the culture after the subculture (that is, 72 hours after the passage through the mesh) to perform medium replacement.

<Evaluation of Subculture>

[Sizes of Spheres before Subculture]

A part of the sphere suspension prepared for subculture in the above-described [Subculture of hiPS Cells] was returned to the wells, and the wells were moved vertically and horizontally on a plane to uniformly disperse the spheres in the wells. The spheres in the wells were observed with a phase contrast microscope (IX73 of Olympus Corporation), and a microscopic image of magnification of 10 times was imaged. The randomly selected 300 sphere images were analyzed, and the circle-equivalent diameters were determined from the area of each individual sphere image to obtain an average of 300 sphere images. The average circle-equivalent diameter was 263.4 μm.

[Sizes and Shapes of Spheres after Subculture]

1 hour after the subculture (that is, 1 hour after the passage through the mesh), the 6-well culture dish was taken out from the incubator, and the wells were moved vertically and horizontally on a plane to uniformly disperse the spheres in the wells. The spheres in the wells were observed with a phase contrast microscope (IX73 of Olympus Corporation), and a microscopic image of magnification of 10 times was imaged. The randomly selected 300 sphere images were analyzed, and the circle-equivalent diameters were determined from the area of each individual sphere image to obtain an average of 300 sphere images. In addition, the spheres were classified by the circle-equivalent diameter as described below, and the ratio "X/Y" of X and Y was obtained.

X: Number of spheres having circle-equivalent diameter of greater than or equal to 30 μm and less than 40 μm.
Y: Number of spheres having circle-equivalent diameter of greater than or equal to 40 μm and less than 300 μm.

The shapes of the spheres were classified as follows from the microscopic image of the spheres.

Classification A: The outline of a sphere is clear and smooth, and the sphere has a spherical shape.
Classification D: The outline of a sphere is not clear and is uneven, and the sphere has an irregular shape.
Classification E: Spheres are excessively divided by subculture, and the sizes of the spheres are inadequately reduced.

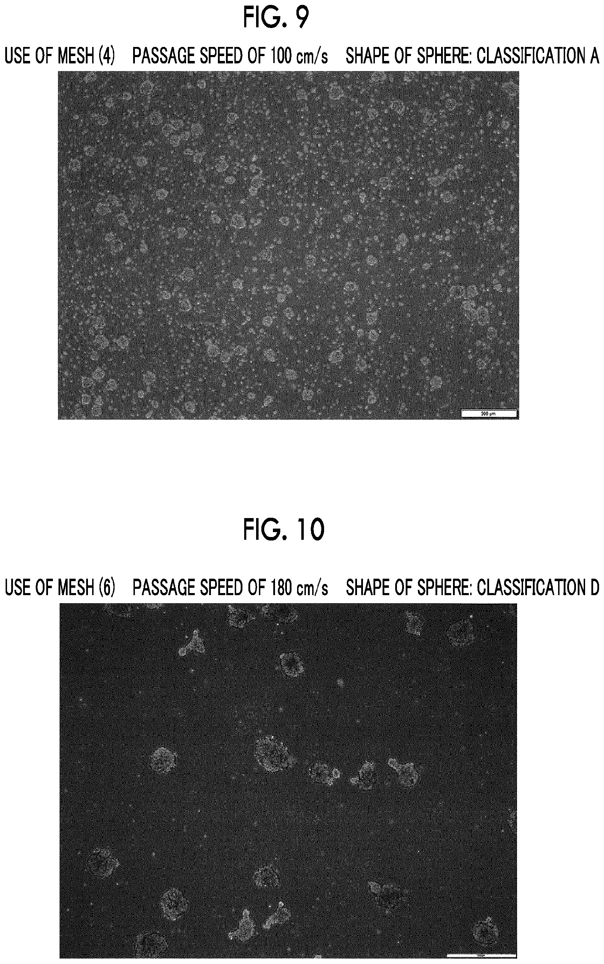

Fig. 9 is a sphere image after spheres are subcultured by being made to pass through a mesh (4) at a speed of 100 cm/s, and is a typical example of the shapes of the spheres of the classification A.

Fig. 10 is a sphere image after spheres are subcultured by being made to pass through a mesh (6) at a speed of 180 cm/s, and is a typical example of the shapes of the spheres of the classification D.

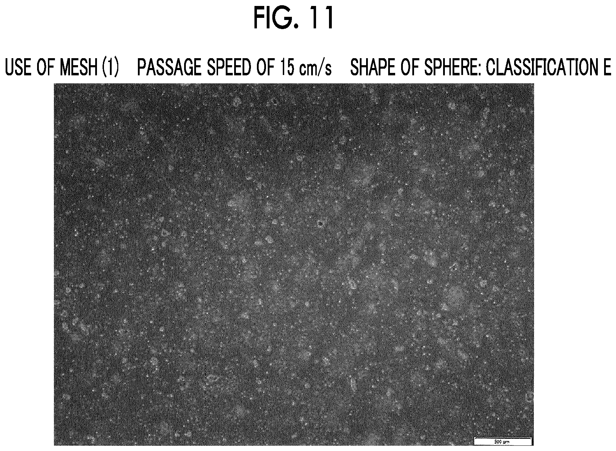

Fig. 11 is a sphere image after spheres are subcultured by being made to pass through a mesh (1) at a speed of 15 cm/s, and is a typical example of the shapes of the spheres of the classification E.

[Cell Collection Rate during Subculture and Cell Proliferation Rate after 5 Days from Subculture]

Immediately before the start of the subculture operation, 1 hour after the subculture (that is, 1 hour after the passage through the mesh), and on day 5 of the culture after the subculture (that is, 120 hours after the passage through the mesh), the 6-well culture dish was taken out from the incubator, and the wells were moved vertically and horizontally on a plane to uniformly disperse the spheres in the wells. 300 μL of a sphere dispersion liquid was placed in a 1 mL tube, 700 μL of TeSR-E8 was added thereto, and a supernatant was removed by centrifugal processing (4,000 rpm, 3 minutes). Subsequently, 300 μL of TrypLE Select (trypsin-like enzyme, model number 12563 of Gibco) was added thereto, and the mixture was stirred with a vortex mixer. The mixture was stirred again with a vortex mixer after being left to stand for 3 minutes at 37°C, and a cell suspension was obtained. The number of cells was measured by applying the cell suspension to a cell counting device (NC-200 of ChemoMetec), the cell collection rate was calculated according to Equation (2), and the cell proliferation rate on day 5 after the subculture was calculated according to Equation (3).

$$\text{Equation (2): Cell collection rate during subculture} = \text{number of cells 1 hour after}$$
$$\text{subculture / number of cells before subculture}$$

$$\text{Equation (3): Cell proliferation rate on day 5 after subculture} = \text{number of cells on}$$
$$\text{day 5 after subculture / number of cells 1 hour after subculture}$$

[Comprehensive Determination]

Classification G1: X/Y is greater than 1 and less than 3, the shapes of spheres are as classification A, the cell collection rate during subculture is greater than or equal to 0.40, and the cell proliferation rate on day 5 after the subculture was greater than or equal to 3.0.
Classification G2: It is the same as classification G1 except that X/Y is greater than 3.
Classification NG: Not applicable to classification G1 and classification G2.

[Table 1]

| Mesh (1) | Speed of passage through mesh | | |
|---|---|---|---|
| | 10 cm/s | 15 cm/s | 50 cm/s |
| Average diameter [μm] of spheres after subculture | 29.2 | 21.0 | 19.6 |
| X/Y | 2.0 | 1.5 | 1.4 |
| Shape of sphere after subculture | E | E | E |
| Cell collection rate during subculture | 0.34 | 0.41 | 0.42 |
| Cell proliferation rate on day 5 after subculture | 1.3 | 1.8 | 1.4 |
| Comprehensive determination | NG | NG | NG |

[Table 2]

| Mesh (2) | Speed of passage through mesh | | | | | |
|---|---|---|---|---|---|---|
| | 10 cm/s | 15 cm/s | 50 cm/s | 100 cm/s | 150 cm/s | 180 cm/s |
| Average diameter [μm] of spheres after subculture | 45.0 | 38.9 | 35.4 | 34.5 | 35.5 | 32.6 |

(continued)

| Mesh (2) | Speed of passage through mesh | | | | | |
|---|---|---|---|---|---|---|
| | 10 cm/s | 15 cm/s | 50 cm/s | 100 cm/s | 150 cm/s | 180 cm/s |
| X/Y | 2.2 | 1.9 | 1.2 | 1.3 | 1.4 | 0.8 |
| Shape of sphere after subculture | A | A | A | A | A | D |
| Cell collection rate during subculture | 0.36 | 0.44 | 0.48 | 0.47 | 0.47 | 0.50 |
| Cell proliferation rate on day 5 after subculture | 2.1 | 3.5 | 3.5 | 4.5 | 4.0 | 2.4 |
| Comprehensive determination | NG | G1 | G1 | G1 | G1 | NG |

[Table 3]

| Mesh (3) | Speed of passage through mesh | | | | | |
|---|---|---|---|---|---|---|
| | 10 cm/s | 15 cm/s | 50 cm/s | 100 cm/s | 150 cm/s | 180 cm/s |
| Average diameter [$\mu$m] of spheres after subculture | 58.8 | 54.1 | 51.2 | 47.1 | 46.7 | 44.4 |
| X/Y | 2.7 | 2.2 | 2.5 | 2.5 | 2.4 | 0.8 |
| Shape of sphere after subculture | A | A | A | A | A | D |
| Cell collection rate during subculture | 0.13 | 0.52 | 0.50 | 0.51 | 0.54 | 0.63 |
| Cell proliferation rate on day 5 after subculture | 2.8 | 5.6 | 5.2 | 5.2 | 5.4 | 2.1 |
| Comprehensive determination | NG | G1 | G1 | G1 | G1 | NG |

[Table 4]

| Mesh (4) | Speed of passage through mesh | | | | | |
|---|---|---|---|---|---|---|
| | 10 cm/s | 15 cm/s | 50 cm/s | 100 cm/s | 150 cm/s | 180 cm/s |
| Average diameter [$\mu$m] of spheres after subculture | 66.3 | 55.6 | 54.9 | 50.4 | 50.5 | 47.9 |
| X/Y | 3.0 | 2.7 | 2.4 | 1.9 | 1.8 | 0.9 |
| Shape of sphere after subculture | A | A | A | A | A | D |
| Cell collection rate during subculture | 0.20 | 0.47 | 0.58 | 0.57 | 0.61 | 0.68 |
| Cell proliferation rate on day 5 after subculture | 4.1 | 5.0 | 5.3 | 6.6 | 6.2 | 1.9 |
| Comprehensive determination | NG | G1 | G1 | G1 | G1 | NG |

[Table 5]

| Mesh (5) | Speed of passage through mesh | | | | | |
|---|---|---|---|---|---|---|
| | 10 cm/s | 15 cm/s | 50 cm/s | 100 cm/s | 150 cm/s | 180 cm/s |
| Average diameter [$\mu$m] of spheres after subculture | 86.0 | 74.1 | 72.2 | 73.3 | 69.3 | 62.6 |
| X/Y | 3.6 | 3.2 | 3.1 | 2.6 | 2.6 | 0.9 |
| Shape of sphere after subculture | A | A | A | A | A | D |
| Cell collection rate during subculture | 0.33 | 0.49 | 0.57 | 0.68 | 0.61 | 0.53 |
| Cell proliferation rate on day 5 after subculture | 2.2 | 5.7 | 6.4 | 6.3 | 6.6 | 2.5 |
| Comprehensive determination | NG | G2 | G2 | G1 | G1 | NG |

[Table 6]

| Mesh (6) | Speed of passage through mesh | | | | | |
|---|---|---|---|---|---|---|
| | 10 cm/s | 15 cm/s | 50 cm/s | 100 cm/s | 150 cm/s | 180 cm/s |
| Average diameter [$\mu$m] of spheres after subculture | 122.7 | 124.4 | 133.1 | 132.1 | 119.4 | 111.0 |
| X/Y | 6.3 | 5.7 | 4.5 | 3.2 | 3.3 | 3.3 |
| Shape of sphere after subculture | D | D | D | D | D | D |
| Cell collection rate during subculture | 0.45 | 0.55 | 0.64 | 0.58 | 0.60 | 0.64 |
| Cell proliferation rate on day 5 after subculture | 1.3 | 1.1 | 1.3 | 1.4 | 1.2 | 0.9 |
| Comprehensive determination | NG | NG | NG | NG | NG | NG |

By allowing the spheres of pluripotent stem cells to pass through a mesh having an opening dimension in a range of 30 $\mu$m to 80 $\mu$m at a passage speed of 15 cm/s to 150 cm/s as shown in Tables 1 to 6, it is possible to efficiently divide cell aggregations while suppressing damage to pluripotent stem cells. That is, it can be said that the method for subculturing pluripotent stem cells according to the exemplary embodiment of the present disclosure is a subculture method suitable for mass culture.

**Claims**

1. A method for subculturing pluripotent stem cells, comprising:

    a culture step of culturing pluripotent stem cells to obtain a cell aggregation; and
    a dividing step of dividing the cell aggregation by passing the cell aggregation through a mesh-like film, which has a plurality of through-holes each having an opening dimension of 30 $\mu$m to 80 $\mu$m, at a speed of 15 cm/sec to 150 cm/sec.

2. The subculture method according to claim 1, wherein, in the culture step, the pluripotent stem cells are cultured in a culture liquid containing a polymer compound.

3. The subculture method according to claim 1 or 2, wherein, in the dividing step, an average value of circle-equivalent diameters of divided cell aggregations becomes 30 $\mu$m to 75 $\mu$m.

4. The subculture method according to any one of claims 1 to 3, wherein the pluripotent stem cell is an ES cell or an iPS cell.

**Patentansprüche**

1. Verfahren zur Subkultivierung pluripotenter Stammzellen, aufweisend:

    einen Kultivierungsschritt des Kultivierens pluripotenter Stammzellen, um eine Zellenaggregation zu erhalten; und
    einen Vereinzelungsschritt des Vereinzeinens der Zellenaggregation durch Passieren der Zellenaggregation durch eine netzartige Folie, die eine Mehrzahl durchgehender Öffnungen hat, von denen jede eine Öffnungsabmessung von 30 $\mu$m bis 80 $\mu$m hat, mit einer Geschwindigkeit von 15 cm/sek bis 150 cm/sek.

2. Subkultivierungsverfahren nach Anspruch 1, wobei die pluripotenten Stammzellen in dem Kultivierungsschritt in einer Kulturflüssigkeit, die eine Polymerverbindung enthält, kultiviert werden.

3. Subkultivierungsverfahren nach Anspruch 1 oder 2, wobei in dem Vereinzelungsschritt ein Durchschnittswert der Kreisäquivalentdurchmesser vereinzelter Zellenaggregationen 30 $\mu$m bis 75 $\mu$m wird.

4. Subkultivierungsverfahren nach einem der Ansprüche 1 bis 3, wobei die pluripotente Stammzelle eine ES-Zelle oder eine iPS-Zelle ist.

**Revendications**

1. Procédé de sous-culture de cellules souches pluripotentes, comprenant :

   une étape de culture pour cultiver des cellules souches pluripotentes afin d'obtenir une agrégation de cellules, et
   une étape de division pour diviser l'agrégation de cellules en faisant passer l'agrégation de cellules par un film à quadrillage, lequel présente une pluralité de trous traversants présentant chacun une dimension d'ouverture allant de 30 µm à 80 µm, à une vitesse de 15 cm/s jusqu'à 150 cm/s.

2. Procédé de sous-culture selon la revendication 1, dans lequel lors de l'étape de culture, les cellules souches pluripotentes sont cultivées dans un liquide de culture contenant un composé polymère.

3. Procédé de sous-culture selon la revendication 1 ou 2, dans lequel lors de l'étape de division, une valeur moyenne de diamètres équivalents au cercle d'agrégations de cellules divisées passe de 30 µm à 75 µm.

4. Procédé de sous-culture selon l'une quelconque des revendications 1 à 3, dans lequel la cellule souche pluripotente est une cellule ES (cellule souche embryonnaire) ou une cellule iPS (cellule souche pluripotente induite).

## FIG. 1A

## FIG. 1B

FIG. 2A

IMMEDIATELY AFTER DIVISION

# FIG. 2B

AFTER LAPSE OF 1 HOUR FROM DIVISION

EP 3 453 753 B1

FIG. 2C

AFTER LAPSE OF 2 HOURS FROM DIVISION

# FIG. 2D

## AFTER LAPSE OF 3 HOURS FROM DIVISION

# FIG. 2E

AFTER LAPSE OF 4 HOURS FROM DIVISION

EP 3 453 753 B1

# FIG. 2F

AFTER LAPSE OF 24 HOURS FROM DIVISION

# FIG. 3

# FIG. 4

CULTURE START PROCESSING

# FIG. 5

MEDIUM REPLACEMENT PROCESSING

# FIG. 6

DIVIDING PROCESSING

# FIG. 7

FREEZING PROCESSING

# FIG. 8

```
        ┌─────────────┐
        │    START    │
        └──────┬──────┘
               │                            ╱S101
        ┌──────┴───────────────────┐
        │  CULTURE START PROCESSING │
        └──────┬───────────────────┘
               │
   ┌──────────→│                            ╱S102
   │    ┌──────┴───────────────────┐
   │    │  (FIRST) MEDIUM REPLACEMENT│
   │    │       PROCESSING          │
   │    └──────┬───────────────────┘
   │           │                            ╱S103
   │    ┌──────┴───────────────────┐
   │    │  (SECOND) MEDIUM REPLACEMENT│
   │    │       PROCESSING          │
   │    └──────┬───────────────────┘
   │           │                            ╱S104
   │    ┌──────┴───────────────────┐
   │    │     DIVIDING PROCESSING   │
   │    └──────┬───────────────────┘
   │           │                            ╱S105
   │        ╱──┴──╲
   │   N  ╱  HAS    ╲
   └─────╱ NUMBER OF  ╲
        ╲  CYCLES     ╱
         ╲ REACHED   ╱
          ╲ ... ╱
            │ Y                              ╱S106
        ┌───┴──────────────────┐
        │  FREEZING PROCESSING  │
        └───┬──────────────────┘
            │
        ┌───┴───┐
        │  END  │
        └───────┘
```

HAS NUMBER OF CYCLES REACHED PREDETERMINED NUMBER?

# FIG. 9

USE OF MESH (4)   PASSAGE SPEED OF 100 cm/s   SHAPE OF SPHERE: CLASSIFICATION A

# FIG. 10

USE OF MESH (6)   PASSAGE SPEED OF 180 cm/s   SHAPE OF SPHERE: CLASSIFICATION D

# FIG. 11

USE OF MESH (1)　PASSAGE SPEED OF 15 cm/s　SHAPE OF SPHERE: CLASSIFICATION E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013077423 A
- WO 2014136581 A
- JP 4705473 B
- JP 4223961 B

**Non-patent literature cited in the description**

- **TOMOMI G. OTSUJI et al.** A 3D sphere culture system containing functional polymers for large-scale human pluripotent stem cell production. *stem cell reports,* 01 May 2014, vol. 2 (5), 734-745
- *Nature Protocols,* 2012, vol. 7, 2029-2040